# EUROPEAN PATENT APPLICATION

(11) **EP 1 602 335 A1**
(43) Date of publication of application: **07.12.2005**
(21) Application number: 05011488.3
(22) Date of filing: 27.05.2005
(51) Int. Cl.: A61B 17/122

(54) **Surgical clip with enhanced gripping arrangement**

(30) Priority: 02.06.2004 US 576332 P; 25.03.2005 US 88752
(71) Applicant: Microline Pentax Inc., Beverly MA 01915 (US)
(72) Inventor: Theroux, Marc, MA Grafton 01519 (US); Menn, Pavel, Marblehead MA 01945 (US); Tatterfield, William, Ipswich MA 01938 (US)
(74) Representative: Schaumburg, Thoenes, Thurn, Landskron

(57) **Abstract**

A surgical clip (20) includes a connecting member (32) having a two ends and a pair of legs (24,26). Each leg extends from one end of the connecting member (32). A generally diamond-shaped pattern (44,45) is formed on a portion of the inner surface (40) of at least one of the connecting member (32) and the pair of legs (24,26). The generally diamond-shaped pattern is formed as the result of generally perpendicular diagonal grooves.

## Description

### CROSS-REFERENCE TO RELATED APPLICATION

This application claims the benefit of U.S. Provisional Application No. 60/576,332 filed on June 2, 2004, the disclosure of which is expressly incorporated by reference in its entirety.

### Clip With Enhanced Gripping Arrangement

### BACKGROUND OF THE INVENTION

### 1. Field of the Invention

This invention relates generally to surgical ligation clips for securing body tissues, in which the surgical ligation clips have enhanced gripping properties. The disclosure of U.S. Patent Application No. 29/206,622 filed on June 1, 2004 is expressly incorporated by reference in its entirety.

### 2. Description of Background Information

Surgical clips are utilized in surgical procedures to bind tissue together, for instance, to close body tissues or to hold body tissues in place for a period of time.

It is imperative that surgical clips remain in place and do not move in an undesirable manner, fall out, or otherwise become loose. Existing surgical clips utilized today may have a serration or an indentation to create some degree of friction with the body tissues to be secured. Unfortunately, existing surgical clips have been known to loosen and become insecure in the body tissues. Of course, this type of occurrence may have severe consequences for the patient.

Therefore, it would be advantageous to provide a surgical clip that overcomes the disadvantages of the prior art by providing a surgical clip having an enhanced gripping surface on at least a portion of the surgical clip.

### SUMMARY OF THE INVENTION

It is a feature of the present invention to provide a clip, for example, a surgical clip utilized to hold and secure body tissue. The surgical clip has an enhanced gripping surface on at least a portion of the inner surface of the surgical clip to provide additional engagement of tissues.

According to one feature of the present invention, a clip is provided with a gripping surface, in which the clip is generally U-shaped, and has a generally V-shaped bridging member, and a pair of leg members, in which each leg member extends from opposed ends of the generally V-shaped bridging member, and in which at least a portion of an inner surface of the pair of leg members and at least a portion of an inner surface of the bridging member have a diagonally-knurled pattern thereon. The knurled pattern includes multiple criss-crossed grooves. The knurled pattern is formed on at least a portion of a side wall of the legs. The knurled pattern may be formed from a plurality of protuberances and/or indentations. Further, the knurled pattern may include multiple grooves forming a multiple diamond-shaped grooves, wherein each of the diamond-shaped grooves have a long axis and a short axis. The short axis of the diamond-shaped grooves may be parallel with a longitudinal axis of the leg member or the long axis of the diamond-shaped grooves may be parallel with a longitudinal axis of the leg member. In any event, the diamond-shaped grooves may be formed as indentations or the diamond-shapes may be protuberances.

Another feature of the present invention is to provide a surgical clip that includes a connecting member that has two ends, a pair of legs, in which each leg extends from a respective end of the connecting member, and multiple generally diamond-shaped patterns formed on at least a portion of the inner surface of at least one of the connecting member and the pair of legs. The plurality of generally diamond-shaped patterns may be recessed or raised. The plurality of generally diamond-shaped patterns may be formed on the entire inner surface of the connecting member and the pair of legs. Further, the plurality of generally diamond-shaped patterns may be formed on at least a part of a sidewall of the legs. Still further, each of the plurality of generally diamond shaped patterns have a long axis and a short axis. The short axis of the plurality of generally diamond shaped patterns may be parallel with a longitudinal axis of the leg and/or the long axis of the plurality of generally diamond shaped patterns is parallel with a longitudinal axis of said leg. Yet further, the connecting member may be generally V-shaped. Further, the generally diamond-shaped pattern may be formed from generally perpendicular diagonal grooves.

According to another feature of the present invention, a surgical clip is provided that includes a connecting member that has two ends, a pair of legs, in which each leg extends from a respective end of the connecting member, and multiple intersecting grooves formed on a portion of the inner surface of at least one of the connecting member and the pair of legs. The multiple intersecting grooves are generally perpendicular.

Other exemplary embodiments and advantages of the present invention may be ascertained by reviewing the present disclosure and the accompanying drawings.

### BRIEF DESCRIPTION OF THE DRAWINGS

The present invention is further described in the detailed description which follows, in reference to the noted plurality of drawings by way of non-limiting examples of certain embodiments of the present invention, in which like numerals represent like elements through the several views of the drawings, and wherein:
Figure 1 is a perspective view of a surgical clip having an enhanced tissue engaging surface, according to first embodiment of the present invention;
Figure 2 is a plan view of the surgical clip shown in Figure 1, according to an aspect of the present invention;
Figure 3 is a sectional view of the surgical clip as shown in Figure 2, taken along the lines A-A, according to an aspect of the present invention;
Figure 4 is an outer side view of the surgical clip shown in Figure 1, according to an aspect of the present invention;
Figure 5 is an end view of the surgical clip as shown in Figure 2, taken along the lines B-B, according to an aspect of the present invention;
Figure 6 is a bottom view of the surgical clip shown in Figure 1, according to an aspect of the present invention;
Figure 7 is a perspective view of a surgical clip in which an enhanced tissue engaging surface is provided on only a portion of the inner surface of the clip, according to an aspect the present invention;
Figure 8 is a perspective view of a leg section of the surgical clip having a knurl thereon, according to a second embodiment of the present invention;
Figure 9 is a perspective view of a knurled pattern on a leg portion of the surgical clip, according to third embodiment of the present invention;
Figure 10 is a plan view of a knurled portion of a surgical clip, according to a fourth embodiment of the present invention;
Figure 11 is a perspective view of the surface of the surgical clip shown in Figure 10, according to an aspect of the present invention;
Figure 12 is a perspective view of a portion of the leg of the surgical clip, according to a fifth embodiment of the present invention;
Figure 13 is a perspective view of an arrangement of criss-crossed shaped depressions arranged on the inner surface of the leg of the surgical clip, according to a sixth embodiment of the present invention;
Figure 14 is a plan view a criss-crossed array of diamond shaped faces arranged on the inner side of the surgical clip, according to a seventh embodiment of the present invention;
Figure 15 is a view of the criss-crossed array of diamond shaped faces arranged on the inner surface of the surgical clip as shown in Figure 14, taken along the lines C-C, according to an aspect of the present invention;
Figure 16 is a plan view of an array of diamond shaped depressions arranged on the inner surface of the surgical clip, according to a eighth embodiment of the present invention;
Figure 17 is a view of the array of diamond shaped depressions arranged on the inner surface of the surgical clip as shown in Figure 16 taken along the lines D-D, according to an aspect of the present invention; and
Figure 18 is a perspective view of a knurled pattern on a leg of a surgical clip showing the knurled pattern extending at least a portion of the way around the side portions of the wire forming the surgical clip, according to an aspect of the present invention.

### DETAILED DESCRIPTION OF THE PREFERRED EMBODIMENTS

The particulars shown herein are by way of example and for purposes of illustrative discussion of the embodiments of the present invention only and are presented in the cause of providing what is believed to be the most useful and readily understood description of the principles and conceptual aspects of the present invention. In this regard, no attempt is made to show structural details of the present invention in more detail than is necessary for the fundamental understanding of the present invention, the description is taken with the drawings making apparent to those skilled in the art how the forms of the present invention may be embodied in practice.

The present invention relates to surgical clips used in the occlusion or ligation of body tissues such as blood vessels, lymphatic vessels and combinations thereof, which may be embedded in fat, muscle, fascia, as well as visceral organs of the body. The surgical clips are suitable for use in conventional surgical operations, as well as other more advanced surgical procedures such as laparoscopic surgeries.

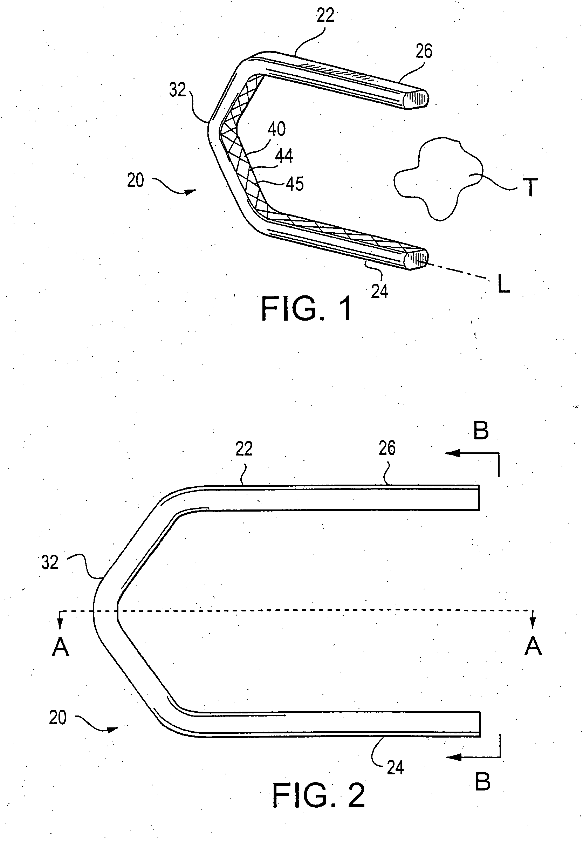

Figure 1 shows a perspective view of a surgical clip, according to a first embodiment of the present invention. The surgical clip 20 is provided to hold and secure body tissues "T", including but not limited to the variety of tissues discussed above. An exemplary surgical clip of the present invention may be approximately one-quarter to one-half centimeter in length, although other lengths are possible, depending upon the specific application.

Figure 2 is a plan view of the surgical clip shown in Figure 1, according to an aspect of the present invention. Referring to Figures 1 and 2, the surgical clip 20 is formed from a generally U-shaped wire 22 having a pair of legs 24 and 26, each of which extends from one end of a generally V-shaped bridging (i.e., connecting) portion 32. Thus, the surgical clip 20 is open at a first end and closed, or connected, at a second end by the bridging portion 32.

The wire 22 forming the legs 24 and 26 is generally rectangular in cross-section as shown, for example in Figures 1, 3 and 4, and has slightly rounded upper and lower surfaces thereon. However, it is noted that any suitable cross-sectional shape may be used without departing from the spirit of the present invention. In this regard, while the term wire is used, it is understood that any suitable stock may be used. Further, it is recognized that the surgical clip 20 may be formed from any suitable material, or combination of materials, including but not limited to metals, composites, plastics, resinous materials, and/or synthetics.

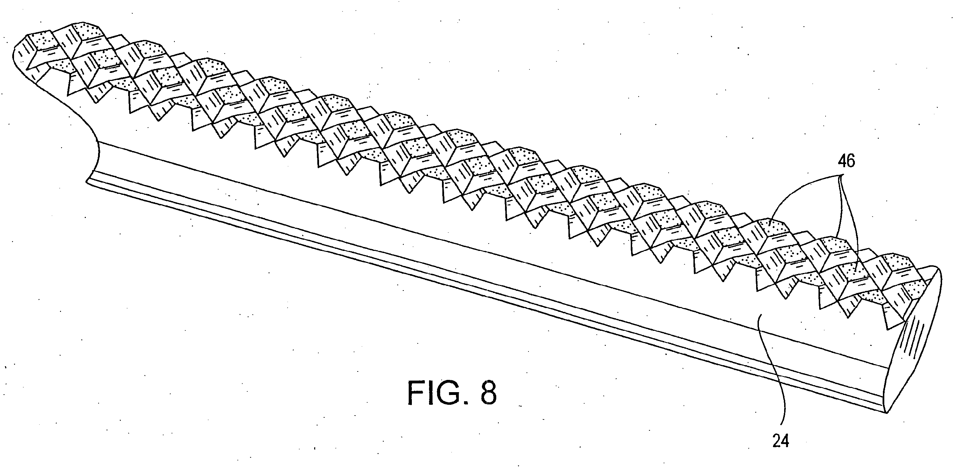

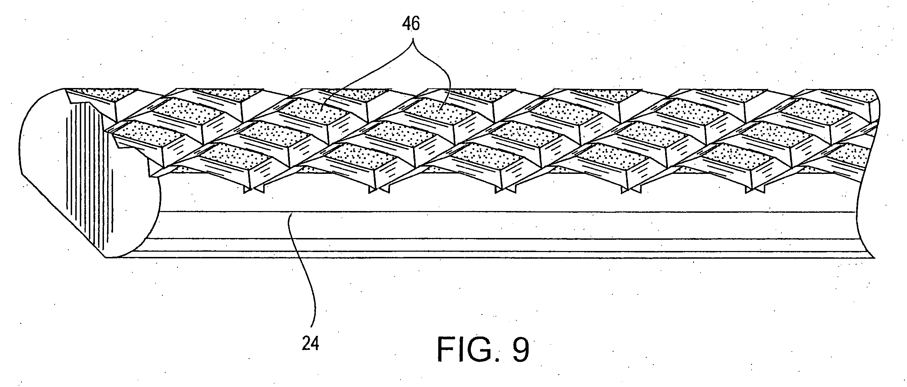

The wire 22 forming the surgical clip 20 has a longitudinal axis "L", as shown in Figure 1. In one embodiment, an inner directed surface 40 of the surgical clip has a surface with a first plurality of spaced apart diagonally arranged grooves, bumps or cuts 44 thereon, as shown for example in Figures 1, 3, and 5. The inner surface 40 also has a second plurality of spaced part diagonally arranged plurality of grooves, bumps or cuts 45, which are generally perpendicular to the first plurality of diagonally-arranged grooves 44. As a result, the first and second pluralities of diagonally-arranged grooves 44 and 45 formed on the inner surface 40 constitute a series of criss-crossing grooves and together form a generally diamond-like pattern of tissue-engaging surfaces 46 on the inner surface 40 of the surgical clip 20, for example, as shown in Figures 8 and 9.

The diagonal criss-crossed grooves provide multiple gripping angles of tissue by the inner surface 40 of the surgical clip 20. The diamond-shaped tissue-engaging surfaces 46 provide acute angles at opposed corners thereof and obtuse angles at their alternate opposing corners, for superior tissue engagement by the surgical clip 20 into the tissue "T". As a result, movement or slippage of the tissue "T" within the surgical clip 20 is minimized.

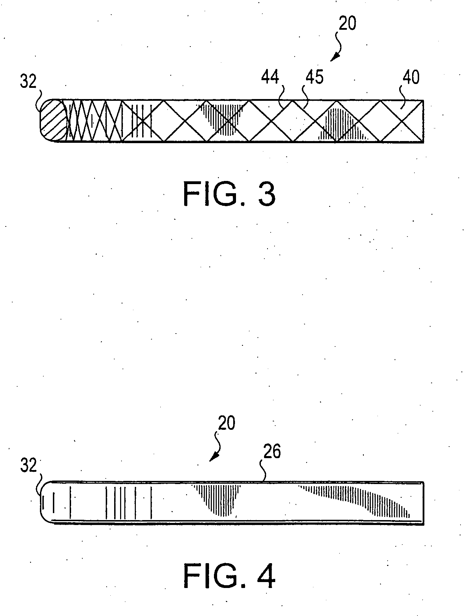

Figure 3 is a sectional view of the surgical clip as shown in Figure 2, taken along the lines A-A. On the inner surface 40 of the surgical clip 20, the first plurality of spaced-apart, diagonally arranged grooves 44 is generally perpendicular to the second plurality of spaced apart diagonally arranged grooves 45. As is shown, the inner surface 40 of the bridging member 32 is also provided with the pluralities of grooves.

Figure 4 is an outer side view of the surgical clip shown in Figure 1. The outer surface of the bridging member 32 and the leg 26 are shown to be free of any grooving in this embodiment. However, in an alternate embodiment, it is possible to provide a similar pattern of grooving as shown in Figure 3, for instance, on at least one of the outer surface of the bridging member and the legs.

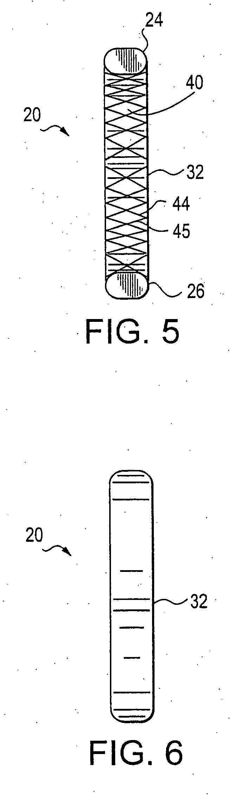

Figure 5 is an end view of the surgical clip as shown in Figure 2, taken along the lines B-B. On the inner surface 40 of the surgical clip 20, the first plurality of spaced apart diagonally-arranged grooves 44 is generally perpendicular to the second plurality of spaced apart diagonally arranged grooves 45. As is shown, the inner surface 40 of the bridging member 32, intermediate of the legs 24 and 26, is also provided with the pluralities of grooves.

Figure 6 is a bottom view of the surgical clip as shown in Figure 1. As is shown, the bridging member 32 is generally rectangular, having rounded edges and corners.

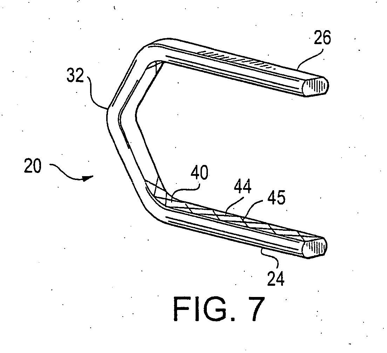

Figure 7 is a perspective view of a surgical clip in which an enhanced tissue engaging surface is provided on only a portion of the inner surface of the clip. As shown, the enhanced tissue engaging surface does not extend around the entire inner surface 40 of the surgical clip 20. In this embodiment, the enhanced tissue-engaging surface extends along the inner surfaces of both legs 24, 26 of the surgical clip 20, and along a portion of the inner surface of the bridging member 32. It should be noted that the partial enhanced tissue engaging surface discussed with respect to Figure 7 may be used with any of the other arrangements and/or patterns discussed herein. In another alternative embodiment, the enhanced tissue engaging surface may extend in a non-continuous intermittent manner along the entire inner surface 40 of the surgical clip 20, in a repeating start and stop pattern. In still another embodiment, the tissue-engaging surface may not extend to the end of the legs 24 and 26.

Figure 8 is a perspective view of a leg section of the surgical clip having a knurl thereon, according to a second embodiment of the present invention. As shown the leg 24 includes a generally diamond-like pattern of tissue engaging surfaces 46, which are formed on the inner surface of the surgical clip. The tissue engaging surfaces, similar to gripping knurls, providing additional strength and surgical clip security, without causing any additional tissue trauma. In addition, parallel and perpendicular comparative pull-of tests have demonstrated that the generally diamond-like pattern of tissue engaging surfaces 46 improve the overall gripping and occlusion forces without damaging tissue surfaces, over conventional surgical clip designs.

Figure 9 is a perspective view of a knurled pattern on a leg portion of the surgical clip, according to third embodiment of the present invention. As shown, the diamond-like pattern of tissue engaging surfaces 46 are more shallow than the diamond-like pattern of tissue engaging surfaces of Figure 8. Further, the diamond-like pattern of tissue engaging surfaces 46 of Figure 9 contain broader face surfaces than the diamond-like pattern of tissue engaging surfaces of Figure 8. In any event, certain variations may be made to the exact shape of the diamond-like pattern of tissue engaging surfaces without departing from the spirit of the present invention.

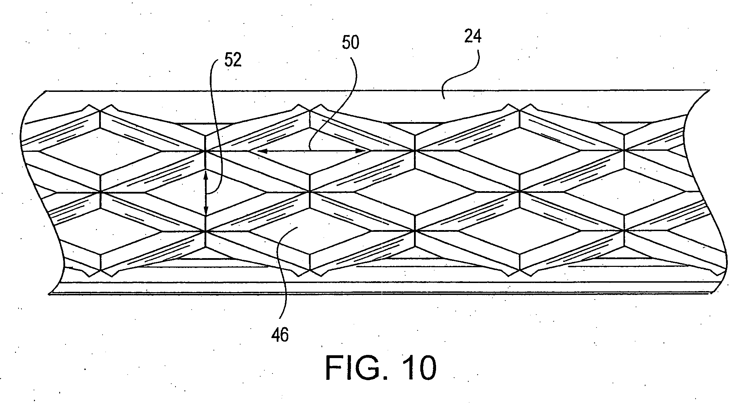

Figure 10 is a plan view of a knurled portion of a surgical clip, according to a fourth embodiment of the present invention. As shown, the enhanced tissue engaging surface 46 has a long axis 50 and a short axis 52. The long axis 50 runs generally parallel with the longitudinal axis "L" of the wire 22 forming the surgical clip 20.

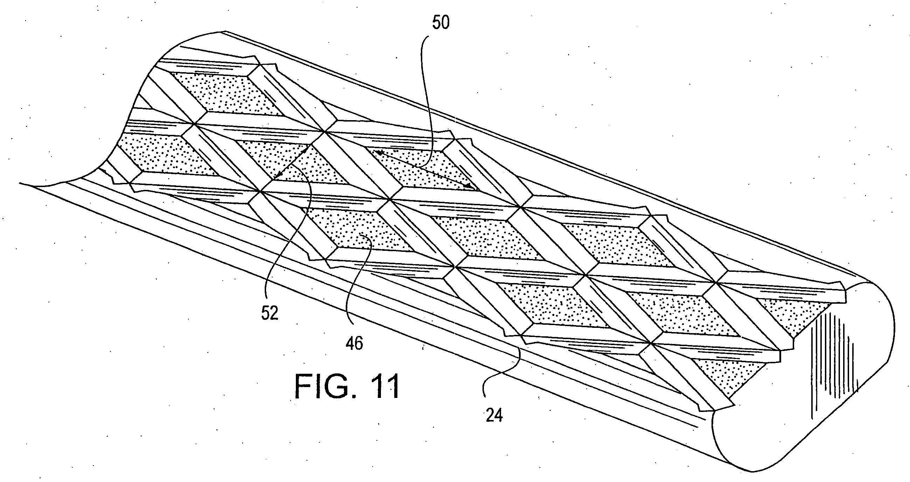

This arrangement may also be seen in Figure 11, which is a perspective view of a surface of the surgical clip shown in Figure 10. The enhanced tissue engaging surface 46 has a long axis 50 and a short axis 52. The long axis 50 runs generally parallel with the longitudinal axis "L" of the wire 22 forming the surgical clip 20.

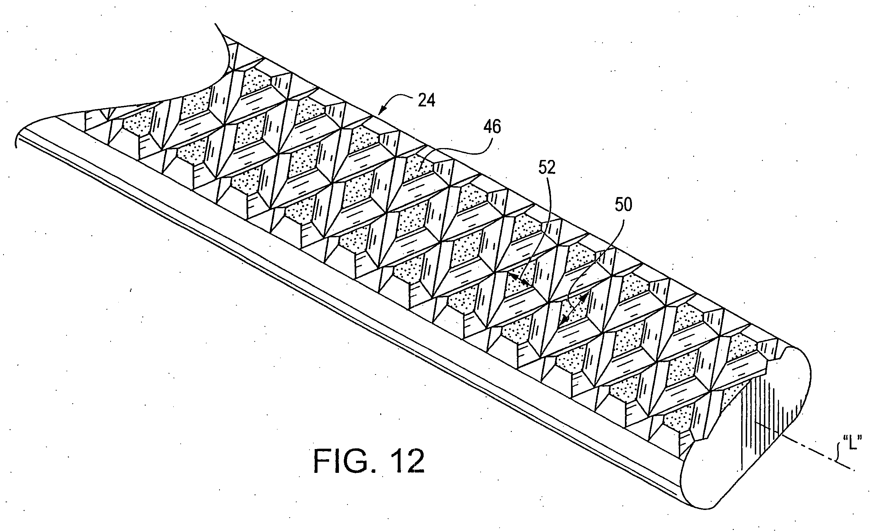

Figure 12 is a perspective view of a portion of the leg of the surgical clip, according to a fifth embodiment of the present invention. As shown, the enhanced tissue engaging surface 46 on the inner surface of the leg 24 has a long axis 50 that runs transversely with respect to the longitudinal axis "L" of the wire 22 of the surgical clip 20.

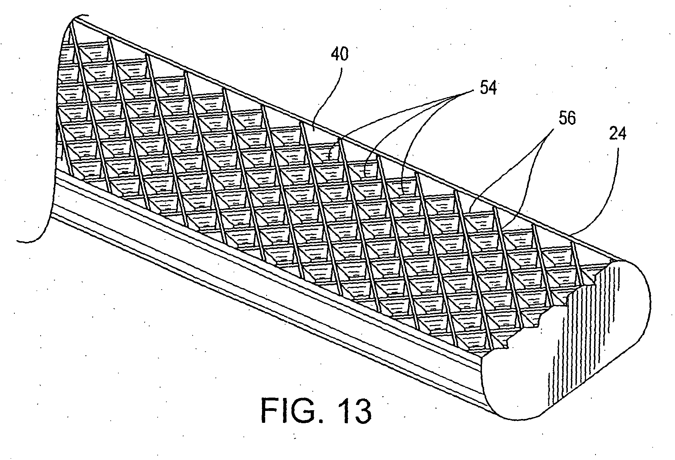

In additional embodiments of the present invention, the generally diamond shaped surfaces reside within depressions or recesses 54 formed in the inner side 40 of the surgical clip 20. Further, criss-crossed ridges 56 may extend upwardly from the diamond shaped surface of the surgical clip 20 as being radially spaced upwardly therefrom, as shown in Figures 13-17.

Figure 13 is a perspective view of an arrangement of criss-crossed shaped depressions arranged on the inner surface of the leg of the surgical clip, according to a sixth embodiment of the present invention. As shown, generally diamond shaped surfaces within depressions or recesses 54 are formed in the inner surface 40 of the surgical clip. Further, criss-crossed ridges 56 extend upwardly from diamond shaped surfaces of the surgical clip 20.

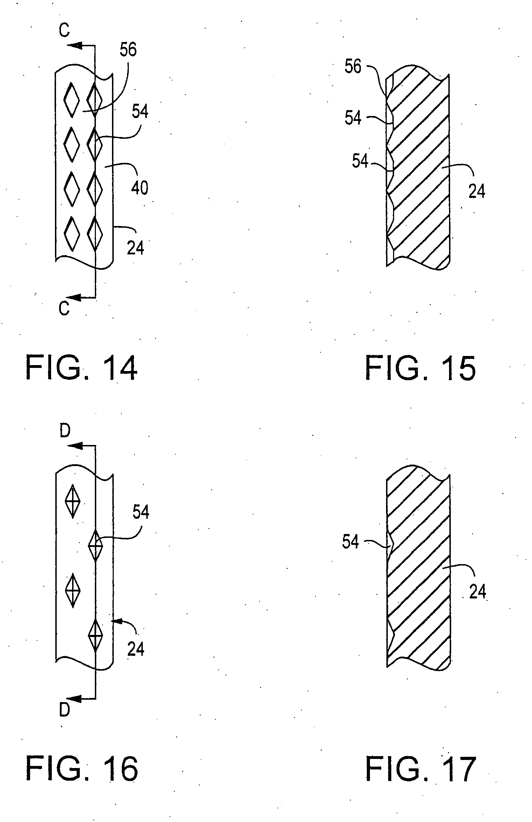

Figure 14 is a plan view a criss-crossed array of diamond shaped faces arranged on the inner side of the surgical clip, according to a seventh embodiment of the present invention. As shown, diamond shaped faces arranged on the inner side of the surgical clip are formed within the recesses 54 in the inner side 40. Figure 15 is a view of the criss-crossed array of diamond shaped faces arranged on the inner surface of the surgical clip as shown in Figure 14, taken along the lines C-C. As is shown, the recesses 54 and the criss-crossed ridges 56 combine to provide additional tissue engagement properties.

Figure 16 is a plan view of an array of diamond shaped depressions arranged on the inner surface of the surgical clip, according to an eighth embodiment of the present invention. As shown, diamond shaped faces arranged on the inner side of the surgical clip are formed within the recesses 54; however, the recesses 54 are arranged in a staggered fashion. Figure 17 is a view of the array of diamond shaped depressions arranged on the inner surface of the surgical clip as shown in Figure 16, taken along the lines D-D. Thus, according to the present invention, the particular arrangement of the diamond shaped patterns discussed herein may be arranged in uniform rows, staggered, or any other suitable arrangement.

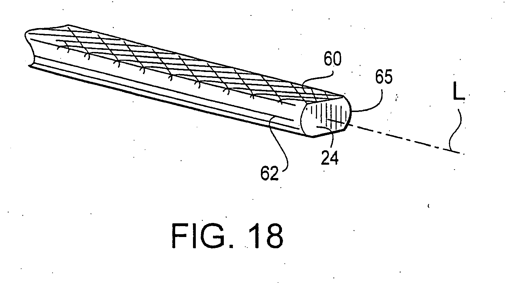

Figure 18 is a perspective view of a knurled pattern on a leg of a surgical clip showing a knurled pattern extending at least a portion of the way around side portions of the wire forming the surgical clip, according to an aspect of the present invention. As shown, an enhanced tissue engaging surface 60 extends at least a portion of the way around the side portions 62 of the wire 65 forming the surgical clip 20. The side portions 62 are knurled, grooved or treated as the inner portion surface 40, which further engages the tissue and further minimizes the likelihood of relevant tissue slippage. In other words, any of the previously described embodiments and/or aspects can be used with the configuration shown in Fig. 18.

The arrangement of diagonally arranged depressions, bumps or protuberances having, in one embodiment, a diamond shape having a longer first axis and a shorter diagonal second axis permits an improved perpendicular pull-off from a tissue, should the surgical clip 20 be removed. The criss-crossed design also permits a better parallel pull-off of the surgical clip 20 should the surgical clip 20 be removed, and provides a superior occlusion of tissue being bound by the surgical clip 20.

Although the invention has been described with reference to an exemplary embodiment, it is understood that the words that have been used are words of description and illustration, rather than words of limitation. Changes may be made, within the purview of the appended claims, as presently stated and as amended, without departing from the scope and spirit of the present invention in its aspects. Although the invention has been described herein with reference to particular means, materials and embodiments, the invention is not intended to be limited to the particulars disclosed herein. Instead, the invention extends to all functionally equivalent structures, methods and uses, such as are within the scope of the appended claims.

## Claims

1. A clip with a gripping surface, the clip comprising a generally U-shaped body comprising:
a generally V-shaped bridging member; and
a pair of leg members, each leg member extending from opposed ends of said generally V-shaped bridging member,
wherein at least a portion of an inner surface of said pair of leg members and at least a portion of an inner surface of said bridging member have a diagonally-knurled pattern thereon.

2. The clip according to claim 1, wherein said knurled pattern comprises a plurality of criss-crossing grooves.

3. The clip according to claim 1, wherein said knurled pattern is formed on at least a portion of a side wall of said legs.

4. The clip according to claim 1, wherein said knurled pattern comprises a plurality of protuberances.

5. The clip according to claim 1, wherein said knurled pattern comprises a plurality of indentations.

6. The clip according to claim 1, wherein said knurled pattern includes a plurality of protuberances and a plurality of indentations.

7. The clip according to claim 1, wherein said knurled pattern comprises a plurality of grooves forming a plurality of diamond shaped grooves.

8. The clip according to claim 7, wherein each of said diamond shaped grooves have a long axis and a short axis.

9. The clip according to claim 8, wherein said short axis of said diamond shaped grooves are parallel with a longitudinal axis of said leg member.

10. The clip according to claim 8, wherein said long axis of said diamond shaped patterns is parallel with a longitudinal axis of said leg member.

11. The clip according to claim 7, wherein said diamond shaped grooves comprise a plurality of indentations.

12. The clip according in claim 4, wherein said plurality of protuberances are diamond shaped.

13. A clip having a gripping surface, comprising:
a connecting member having a two ends;
a pair of legs, each leg extending from a respective end of the connecting member; and
a plurality of generally diamond-shaped patterns formed on at least a portion of the inner surface of at least one of the connecting member and the pair of legs.

14. The clip according in claim 13, wherein the plurality of generally diamond-shaped patterns are recessed.

15. The clip according in claim 13, wherein the plurality of generally diamond-shaped patterns are raised.

16. The clip according in claim 13, wherein the plurality of generally diamond-shaped patterns are formed on the entire inner surface of the connecting member and the pair of legs.

17. The clip according in claim 13, wherein a portion of the plurality of generally diamond-shaped patterns are formed on at least a part of a sidewall of the legs.

18. The clip according in claim 13, wherein the plurality of generally diamond-shaped patterns comprise a plurality of recesses and raised portions.

19. The clip according to claim 13, wherein each diamond shaped pattern of said plurality of generally diamond-shaped patterns have a long axis and a short axis.

20. The clip according to claim 19, wherein said short axis of each said diamond-shaped pattern is generally parallel with a longitudinal axis of said leg.

21. The clip according to claim 19, wherein said long axis of each said diamond-shaped pattern is generally parallel with a longitudinal axis of said leg.

22. The clip according to claim 13, wherein the connecting member is generally V-shaped.

23. The clip according to claim 13, wherein the generally diamond-shaped pattern is formed from generally perpendicular diagonal grooves.

24. A clip having a gripping surface, comprising:
a connecting member having two ends;
a pair of legs, each leg extending from a respective end of the connecting member; and
a plurality of intersecting grooves formed on a portion of the inner surface of at least one of the connecting member and the pair of legs.

25. The clip according to claim 24, wherein the plurality of intersecting grooves are generally perpendicular.
